(19) Europäisches Patentamt
European Patent Office
Office européen des brevets

(11) Veröffentlichungsnummer: **0 221 387**
**A2**

(12) # EUROPÄISCHE PATENTANMELDUNG

(21) Anmeldenummer: 86113949.1

(22) Anmeldetag: 08.10.86

(51) Int. Cl.[4]: C 07 C 179/10
C 07 C 178/00, C 11 D 3/39

(30) Priorität: 04.11.85 DE 3539036

(43) Veröffentlichungstag der Anmeldung:
13.05.87 Patentblatt 87/20

(84) Benannte Vertragsstaaten:
AT BE CH DE ES FR GB IT LI NL SE

(71) Anmelder: Degussa Aktiengesellschaft
Weissfrauenstrasse 9
D-6000 Frankfurt am Main 1(DE)

(71) Anmelder: Henkel Kommanditgesellschaft auf Aktien
Postfach 1100 Henkelstrasse 67
D-4000 Düsseldorf-Holthausen(DE)

(72) Erfinder: Dankowski, Manfred, Dr.
Stifterstrasse 14
D-8757 Karlstein(DE)

(54) **In 2-Stellung substituierte Diperoxyglutarsäuren, ein Verfahren zur Herstellung und ihre Verwendung.**

(57) Die Erfindung betrifft neue in 2-Stellung substituierte Diperoxyglutarsäuren mit einer $H_2O_2$-haltigen Oxidationsmischung erhält. Die neuen Verbindungen eignen sich für die Verwendung bei der Wäsche oder Bleiche von Textilien.

EP 0 221 387 A2

Degussa Aktiengesellschaft, Weißfrauenstraße 9, 6000 Frankfurt am Main
Henkel Kommanditgesellschaft auf Aktien, 4000 Düsseldorf 1

# In 2-Stellung substituierte Diperoxyglutarsäuren, ein Verfahren zur Herstellung und ihre Verwendung

Die Erfindung betrifft in 2-Stellung substituierte Diperoxyglutarsäuren, ein Verfahren zur Herstellung und ihre Verwendung bei der Wäsche oder Bleiche von Textilien, da ihre Wirkung schon bei Temperaturen unterhalb von 80°C eintritt.

Es ist bereits eine Reihe verschiedener Verfahren zur Herstellung von Peroxycarbonsäuren veröffentlicht worden.

Die EP-A-2-0045290 betrifft ein Verfahren zur Herstellung von Peroxycarbonsäuren, bei dem die Ausgangscarbonsäuren in konzentrierter Schwefelsäure gelöst, und die durch Oxidation mit Wasserstoffperoxid gebildeten Persäuren kontinuierlich mit einem organischen Lösungsmittel extrahiert werden.

In der US-PS 4 119 660 wird ebenfalls ein Verfahren beschrieben, bei dem die Ausgangscarbonsäuren in großen Überschüssen von Schwefelsäure gelöst und anschließend zu Persäuren umgesetzt werden.

Die EP-A-1-0083560 betrifft die Herstellung von substituierten Diperoxybernsteinsäuren durch die Umsetzung der entsprechenden, in Methansulfonsäure gelösten Anhydride mit Wasserstoffperoxid.

...

Aufgabe der Erfindung sind besonders wirksame Peroxycarbonsäuren, ihre Herstellung und ihre Verwendung bei der Wäsche oder Bleiche von Textilien.

Gegenstand der Erfindung sind in 2-Stellung substituierte Diperoxyglutarsäuren der Formel

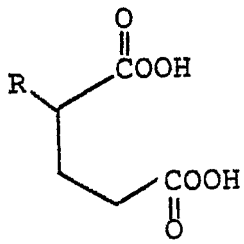

, in der bedeuten

R: $C_3$-$C_{18}$ Alkyl, linear oder verzweigt,
insbesondere $C_5$-$C_{13}$-Alkyl,
$C_3$ bis $C_8$ Cycloalkyl
insbesondere $C_5$ bis $C_6$ - Cycloalkyl,
Aryl, insbesondere Phenyl

Gegenstand der Erfindung ist gleichzeitig ein Verfahren zur Herstellung dieser Verbindungen unter Verwendung einer Oxidationsmischung aus Wasserstoffperoxid, Wasser und Schwefelsäure, das dadurch gekennzeichnet ist, daß man eine Säure der Formel

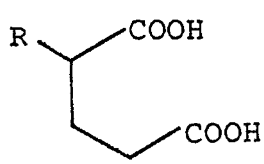

, in der

R die oben angegebenen Bedeutungen hat, bei 0 bis 40°C, bevorzugt 10 - 25°C, in die Oxidationsmischung eindosiert und bei 0 bis 40°C, bevorzugt 15 - 25°C, innerhalb von 1 bis 40h, bevorzugt 3 - 24h unter Rühren umsetzt.

...

Bei den anfallenden Reaktionsgemischen handelt es sich Abhängigkeit von dem Substituenten R um eine Suspension oder eine Lösung oder auch eine Zwischenstufe dieser beiden Möglichkeiten.

In Abhängigkeit davon wählt man das Abtrennverfahren.

In allen Fällen versetzt man nach dem Abschluß der Reaktion das Gemisch unter Kühlung mit der 0.1 bis 10-fachen, bevorzugt 0.7 bis 4-fachen Menge destilliertem Wasser, bezogen auf die Gemischmenge, und extrahiert aus Lösungen mit Essigester die gewünschte Peroxycarbonsäure.

Liegt diese vollständig in gut kristallisierter Form vor, ist die einfache Abtrennung über ein Filter ohne zusätzliche organische Lösungsmittel vorteilhaft.

Die Aufarbeitung erfolgt dann mit den allgemein üblichen Maßnahmen.

Bei der Durchführung des erfindungsgemäßen Verfahrens erweist es sich als günstig, Schwefelsäure und Carbonsäure im Molverhältnis 2 bis 50 zu 1, bevorzugt 3 bis 40 zu 1, einzusetzen.
Die Umsetzung wird bei Temperaturen von 0-40°C, bevorzugt von 15 - 25°C, durchgeführt.
Wasserstoffperoxid wird in Konzentrationen von 20 bis 99 Gew.-%, bevorzugt 40 bis 85 Gew.-%, eingesetzt, Schwefelsäure in Konzentrationen von 20 bis 99 Gew.-%, bevorzugt 90 bis 99 Gew.-%.

Als Molverhältnis von Carbonsäure zu Wasserstoffperoxid wählt man 1 zu 2 bis 1 zu 50, bevorzugt 1 zu 3 bis 1 zu 30.

...

Besonders geeignet sind die 2-Pentylglutarsäure, 2-Hexylglutarsäure, 2-Heptylglutarsäure, 2-Octylglutarsäure, 2-Nonylglutarsäure und die 2-Decylglutarsäure

Die erfindungsgemäßen Diperoxyglutarsäuren erweisen sich als mindestens in gleicher Weise wirksam, vielfach jedoch als überlegen im Vergleich zu dem bekannten Perborat/TAED-Bleichsystem. (TAED: Tetraacetylethylendiamin).

Beispiel 1:

Versuchsdurchführung:

Die Versuche wurden bei T = 40°C im Launder-o-meter unter Einsatz der Testgewebe Rotwein, Kaffee und Tee (sämtlich bleichmittelfrei vorgewaschen) durchgeführt. Die 2-Alkyldiperoxyglutarsäuren wurden so dosiert, daß jeweils eine Aktivsauerstoffkonzentration ($O_a$) von 35 mg/l in der Waschflotte resultierte. In einer weiteren Versuchsreihe wurde Natriumperborat in einer Konzentration von 0,35 g/l zusammen mit 0,25 g/l TAED eingesetzt.

Diese Kombination liefert in der Waschflotte theoretisch 35 mg Oa/l in Form von Peressigsäure (PES). Zur Bestimmung des auswaschbaren Anteils der Fleckenanschmutzungen wurde bleichmittelfrei mit einer Rahmenrezeptur gewaschen (Rezepturübersicht siehe Tab. 1). Die Bleichwirkung wurde als Remissionszunahme an den verschiedenen Testgeweben gemessen. Die Auswertung der Meßergebnisse erfolgte in gewohnter Weise statistisch über die Bestimmung von LSD (least significant difference) und F-Werten.

...

Tabelle 1:

Waschrezeptur

| | | |
|---|---|---|
| Natriumaluminiumsilikat | | 25.0 % |
| Pentanatriumtriphosphat | | 25.0 % |
| Natriumdisilikat | | 6.3 % |
| Magnesiumsilikat | | 5.0 % |
| Natriumsulfat | | 7.5 % |
| Carboxymethylcellulose | | 3.8 % |
| Dequest 2041 | | 0.4 % |
| Tenside, verschied. | | 18.2 % |
| Marlon A 350 | 7.5 % | |
| Marlipal 1618 | 6.3 % | |
| med.Seife Merck | 4.4 % | |

Rest: Füllstoff, Wasser

Dosierung 6g/l

Tabelle 2:

Remissionszunahme verschiedener Testgewebe bei T = 40°C

1. EMPA-Rotwein

AW(Ausgangsremissionswert) = 50,5(%)R

| | ΔR(%) |
|---|---|
| Perborat/TAED | 12.7 |
| 2-Pentyldiperoxyglutarsäure | 13.0 |

2. Tee

AW = 52,3(%)R

| | ΔR(%) |
|---|---|
| Perborat/TAED | 18.7 |
| 2-Pentyldiperoxyglutarsäure | 19.6 |
| 2-Nonyldiperoxyglutarsäure | 19.4 |
| 2-Decyldiperoxyglutarsäure | 19.5 |

3. Kaffee

AW = 63,9(%)R

| | ΔR(%) |
|---|---|
| Perborat/TAED | 15.1 |
| 2-Nonyldiperoxyglutarsäure | 16.4 |
| 2-Undecyldiperoxyglutarsäure | 16.1 |

...

Beispiel 2

2-Pentyl-diperoxyglutarsäure

In eine Oxidationsmischung, bestehend aus 32,6 g (0,48 mol) 50 Gew.-%igem Wasserstoffperoxid, 62,7 g (0,64 mol) 96 Gew.-%iger Schwefelsäure und 30 mg Tri-n-octylphosphanoxid, werden bei 20°C unter Rühren 16,2 g (0,08 mol) 2-Pentyl-glutarsäure eindosiert und anschließend 3h bei Raumtemperatur zur Reaktion gebracht. Danach wird unter Kühlung 200 ml destilliertes Wasser hinzugegeben und viermal mit je 50 ml Essigester extrahiert. Die Lösung wird mit destilliertem Wasser neutral gewaschen, über Natriumsulfat getrocknet, und nach dem Abdampfen des Lösungsmittels werden nach Trocknen bei 30°C im Vakuum 17 g 2-Pentyl-diperoxyglutarsäure erhalten.
Der Gehalt an Persäure beträgt 89,7 Gew.-%
Die Ausbeute beträgt 81 %.

Beispiel 3

2-Nonyl-diperoxyglutarsäure

In eine Oxidationsmischung, bestehend aus 41 g (0,60 mol) 50 Gew.-%igem Wasserstoffperoxid, 78,4 g (0,80 mol) 96 Gew.-%iger Schwefelsäure und 20 mg Tri-n-octylphosphanoxid werden bei 20°C unter Rühren 12,9 g (0,05 mol) 2-Nonyl-glutarsäure eindosiert und anschließend 4h bei Raumtemperatur zur Reaktion gebracht. Danach wird unter Kühlung 300 ml destilliertes Wasser hinzugegeben und viermal mit je 50 ml Essigester extrahiert. Die Lösung wird mit destilliertem Wasser neutral gewaschen, über Natriumsulfat getrocknet, und nach dem Abdampfen des Lösungsmittels werden nach Trocknen bei 30°C im Vakuum 13,8 g 2-Nonyl-diperoxyglutarsäure erhalten.
Der Gehalt an Persäure beträgt 89,2 Gew.-%
Die Ausbeute beträgt 85 %.

...

Beispiel 4

2-Undecyl-diperoxyglutarsäure

In eine Oxidationsmischung, bestehend aus 4,1 g (0,06 mol) 50 Gew.-%igem Wasserstoffperoxid, 7,8 g (0,08 mol) 96 Gew.-%iger Schwefelsäure und 3 mg Tri-n-octylphosphanoxid, werden bei 20°C unter Rühren 2,9 g (0.01 mol) 2-Undecyl-glutarsäure eindosiert und anschließend 20h bei Raumtemperatur zur Reaktion gebracht. Danach wird unter Kühlung 50 ml destilliertes Wasser hinzugegeben und der Reaktionsbrei über eine Nutsche separiert. Nach mehrmaligem Waschen mit destilliertem Wasser wird der Filterrückstand bei 30°C im Vakuum über Blaugel getrocknet. Es werden 2,7 g 2-Undecyl-diperoxyglutarsäure erhalten.

Der Gehalt an Persäure beträgt 92 %.

Die Ausbeute beträgt 85 %.

0221387
85 175 PV
01.11.1985

Degussa Aktiengesellschaft
Weissfrauenstraße 9, 6000 Frankfurt am Main

Henkel Kommanditgesellschaft auf Aktien
4000 Düsseldorf 1

In 2-Stellung substituierte Diperoxyglutarsäuren, ein Verfahren zur Herstellung und ihre Verwendung

## Patentansprüche

1. In 2-Stellung substituierte Diperoxyglutarsäuren der Formel

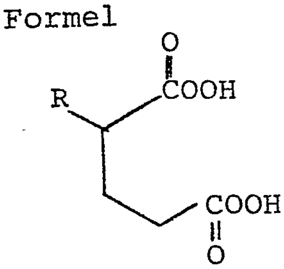

, (I)

in der R bedeutet: ein $C_3$-$C_{18}$ Alkyl, linear oder verzweigt, $C_3$ -$C_8$ Cycloalkyl, Aryl, insbesondere Phenyl

2. Verfahren zur Herstellung von Verbindungen gemäß Anspruch 1 unter Verwendung einer Oxidationsmischung aus Wasserstoffperoxid, Wasser und Schwefelsäure, dadurch gekennzeichnet, daß man Säuren mit der Formel (II)

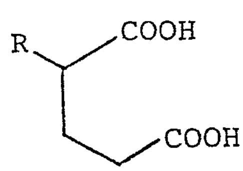

, in der R die

...

oben angegebene Bedeutung hat, bei 0 bis 40°C in die Oxidationsmischung eindosiert, in diesem Temperaturbereich die Reaktion durchführt und anschließend das Produkt in an sich bekannter Weise abtrennt.

3. Verwendung der Verbindungen gemäß den Ansprüchen 1 und 2 in Wasch- und Reinigungsmitteln sowie in reinen Bleichboostern.